Europäisches Patentamt

European Patent Office

Office européen des brevets

(11) Veröffentlichungsnummer: 0 315 096
A2

## (12) EUROPÄISCHE PATENTANMELDUNG

(21) Anmeldenummer: 88118070.7

(22) Anmeldetag: 29.10.88

(51) Int. Cl.4: C07C 67/08 , C07C 69/63

(30) Priorität: 06.11.87 DE 3737759

(43) Veröffentlichungstag der Anmeldung:
10.05.89 Patentblatt 89/19

(84) Benannte Vertragsstaaten:
AT BE CH DE ES FR GB IT LI NL

(71) Anmelder: Henkel Kommanditgesellschaft auf
Aktien
Postfach 1100 Henkelstrasse 67
D-4000 Düsseldorf 1(DE)

(72) Erfinder: Wüst, Willi, Dr.
Fasanenring 32
D-4030 Ratingen(DE)
Erfinder: Leischner, Hasso
Böcklinstrasse 10
D-4000 Düsseldorf 1(DE)
Erfinder: Esser, Herbert
Siglarer Strasse 64
D-5210 Troisdorf(DE)

(54) Verfahren zur Herstellung von Estern der Monochloressigsäure mit C1-C4-Alkanolen.

(57) Ein Verfahren zur Herstellung von Estern der
Monochloressigsäure mit $C_1$-$C_4$-Alkanolen durch
Veresterung der freien Säure mit den Alkoholen in
Gegenwart von Veresterungskatalysatoren, bei dem
der Alkohol der katalysatorhaltigen Schmelze der
Monochloressigsäure unter Entfernung des Reaktionswassers mit solcher Geschwindigkeit zugegeben wird, daß das sich bildende Reaktionswasser
zusammen mit Anteilen des entstandenen Chloressigsäureesters als praktisch alkoholfreies binäres
Azeotrop abdestilliert wird, liefert hohe Ausbeuten
und eine problemlose Abtrennung der gewünschten
Endprodukte.

Fig. 1

EP 0 315 096 A2

## Verfahren zur Herstellung von Estern der Monochloressigsäure mit $C_1$-$C_4$-Alkanolen.

Die Erfindung betrifft ein Verfahren zur Herstellung von Estern der Monochloressigsäure mit $C_1$-$C_4$-Alkanolen durch Veresterung der freien Säure mit den Alkoholen in Gegenwart von Veresterungskatalysatoren.

Ester der Monochloressigsäure mit niederen Alkoholen sind seit langem bekannt. Von besonderer Wichtigkeit sind der Methyl-und der Ethylester als Ausgangsprodukte für die Synthese von Sarkosin, Chloracetamid, Thioglykolsäureester und Heterocyclen, wobei die Bedeutung des Methylesters überwiegt.

Sowohl der Methyl- als auch der Ethylester der Monochloressigsäure können aus Monochloressigsäure durch Veresterung mit Methanol bzw. Ethanol hergestellt werden. Eine weitere Sythesemöglichkeit besteht in der Verseifung der entsprechenden 1,2-Dichlorvinylether.

Aus Przem. Chem. 1982, 61 (10), 386-387, ist die Veresterung von Monochloressigsäure mit Ethanol in 96%-iger Ausbeute bekannt, wobei ein 2,5-fach molarer Überschuß an Ethanol eingesetzt und das gebildete Reaktionswasser als Azeotrop aus dem Reaktionsgemisch entfernt wird.

In der DL-PS 97 416 ist ein Verfahren beschrieben, bei dem in vorgelegtem siedendem Monochloressigsäuremethylester Monochloressigsäure und Methanol in äquimolaren Mengen eingeleitet und umgesetzt werden, wobei über eine Destillationskolonne ein ternäres Azeotrop aus Methanol, Wasser und Chloressigsäuremethylester am Kolonnenkopf und im Unterteil der Kolonne ein binäres Gemisch aus Monochloressigsäuremethylester und Wasser abgenommen werden. Die DL-PS 97 416 erwähnt weiterhin ein Verfahren, bei dem eine Lösung äquimolarer Mengen Monochloressigsäure in dem entsprechenden Alkohol in geschmolzene Monochloressigsäure eingeleitet wird, wobei nicht umgesetztes Methanol als ternäres Azeotrop zusammen mit Monochloressigsäuremethylester und Wasser abdestilliert wird.

Das ternäre Azeotrop von Monochloressigsäureethylester, Ethanol und Wasser siedet unter Normalbedingungen bei 81 °C und weist eine Zusammensetzung von 20,8 Gew.-% Ester, 61,7 Gew.-% Ethanol und 17,5 Gew.-% Wasser auf. Das entsprechende, von dem Methylester gebildete ternäre Azeotrop weist einen höheren Gehalt an Methanol und einen geringeren Gehalt an Wasser auf.

Die Bildung dieser ternären Azeotrope bei den vorgenannten Verfahren ist nachteilig, denn die Azeotrope sind homogen und spalten bei Raumtemperatur nicht in Phasen auf. Dies hat zur Folge, daß zur Wiedergewinnung des Ethanols und zur Isolierung des Wertproduktes Maßnahmen ergriffen werden müssen, die verfahrenstechnisch sehr aufwendig, energieintensiv und abwasserbelastend sind. Die Aufarbeitung kann z.B. in Form einer Extraktion mit anschließender destillativer Trennung von Raffinat und Extrakt erfolgen. Arbeitet man zur Vermeidung dieser Nachteile nach dem obengenannten, in Przem. Chem. 1982 beschriebenen Verfahren und nimmt den Ethanolüberschuß zurück, beobachtet man jedoch eine drastische Abnahme der Ausbeute, so daß dieses Verfahren unbrauchbar wird.

Überraschenderweise wurde nun gefunden, daß sich die obengenannten Nachteile, insbesondere bedingt durch die Ausbildung ternärer Azeotrope, vermeiden lassen, wenn man den Alkohol der katalysatorhaltigen Schmelze der Monochloressigsäure unter Entfernung des Reaktionswassers mit solcher Geschwindigkeit zugibt, daß das sich bildende Reaktionswasser zusammen mit Anteilen des entstandenen Chloressigsäureesters als praktisch alkoholfreies binäres Azeotrop abdestilliert wird.

Gemäß einer vorteilhaften Ausführungsform der Erfindung führt man die Veresterung unter gleichzeitiger Abtrennung der alkoholfreien dampfförmigen wasserhaltigen Phase bei Temperaturen oberhalb von 100 °C, vorzugsweise im Bereich von etwa 120 bis 170 °C und insbesondere im Bereich von etwa 135 bis 160 °C durch.

Gemäß einer weiteren vorteilhaften Ausführungsform der Erfindung arbeitet man im Druckbereich von etwa 0,3 bis 5 bar, insbesondere im Bereich des Normaldrucks; durch Drucksteigerung läßt sich der Wasseranteil im binären Azeotrop erhöhen.

Gemäß einer weiteren vorteilhaften Ausführungsform der Erfindung arbeitet man - bezogen auf die Gesamtumsetzung - mit etwa äquimolaren Mengen an Monochloressigsäure zu $C_{1-4}$- Alkohol, vorzugsweise im Bereich von 1 : 1-1,05.

Besonders vorteilhaft ist es weiterhin, wenn man den Gehalt des Reaktionsgemisches - bei kontinuierlicher Arbeitsweise der nachstehend genannten Art am Kolonnenkopf einer Reaktionskolonne - an freiem Alkohol während der Umsetzung auf Werte von maximal etwa 1 Gew.-% begrenzt.

Gemäß einer weiteren vorteilhaften Ausführungsform der Erfindung kondensiert man das destillativ abgetrennte binäre Azeotrop unter Phasentrennung, trennt die untere Phase als Wertprodukt ab und gewinnt aus der oberen wäßrigen Phase, vorzugsweise durch erneute partielle Destillation, wenigstens anteilsweise die dort enthaltenen Restmengen an Chloressigsäureester.

Das Verfahren der Erfindung kann absatzweise, vorzugsweise jedoch kontinuierlich gemäß den folgenden Verfahrensmaßnahmen durchgeführt werden: Gegenstromführung der katalysatorhaltigen Schmelze der Monochloressigsäure und des Alkohols in einer Reaktionskolonne bei Einspeisung der Schmelze im Kopfbereich, Abziehen des wasserhaltigen binären Azeotrops am Kolonnenkopf, vorzugsweise unter Rückführung eines Anteils des nach Kondensation aus dem Azeotrop gewonnenen Chloressigsäureesters auf den Kolonnenkopf und Gewinnung der Hauptmenge des Chloressigsäureesters am anderen Ende der Reaktionskolonne.

Vorzugsweise führt man den Alkohol in die Schmelzphase nach Vorerhitzung etwa auf Reaktionstemperatur ein. Schließlich wird das Verfahren der Erfindung bevorzugt auf die Herstellung der Methyl- oder Ethylester, insbesondere des Ethylesters der Monochloressigsäure angewendet.

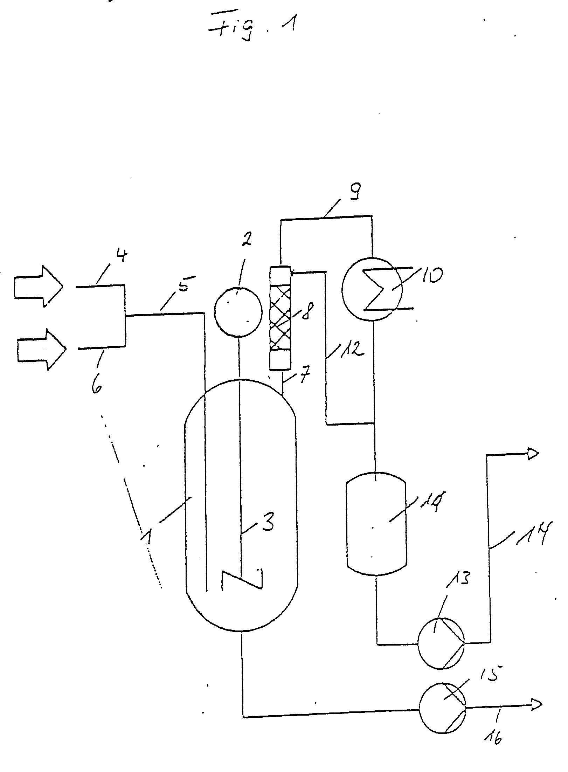

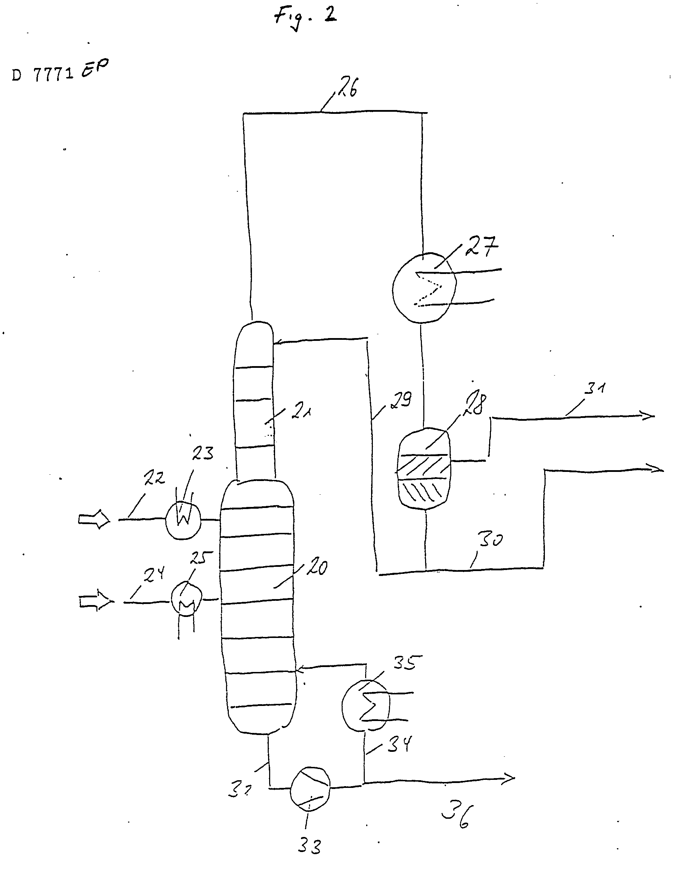

Weitere Verfahrenseinzelheiten ergeben sich aus der folgenden Beschreibung, in der auf die Figuren 1 und 2 Bezug genommen wird. Es zeigen:

Fig. 1 en Fließschema für eine diskontinuierliche Verfahrensführung und

Fig. 2 ein Fließschema für eine kontinuierliche Verfahrensführung.

Gemäß Fig. 1 wird in einem Reaktor 1 mit einem durch einen Motor 2 angetriebenen Rührer 3 Monochloressigsäure mit darin gelösten 0,05 bis 5,00 Mol-% Veresterungskatalysator über Leitungen 4 und 5 vorgelegt und aufgeschmolzen. Die Leitung 5 mündet dabei am bzw. in der Nähe des Bodens des Reaktors 1. Nachdem die Schmelze eine Temperatur von 120 bis 170 °C erreicht hat, wird über eine Leitung 6 und die Leitung 5 Ethanol zudosiert.

Das während der Veresterungsreaktion gebildete, im Gleichgewicht vorhandene Reaktionswaser wird als binäres Azeotrop mit Monochloressigsäurester über eine Leitung 7 und einen Rektifikationsaufsatz 8 sowie eine Leitung 9 mit dazwischengeschaltetem Wärmeaustauscher 10 in einem Absetzbehälter 11 überführt. In dem Absetzbehälter fällt ein zweiphasiges Kondensat an. Die schwere untere Phase besteht praktisch aus reinem Monochloressigsäureester und ist für die Weiterverarbeitung genügend rein. Sie enthält lediglich entsprechend der Sättigungskonzentration je nach Kondensationsbedingungen 0,7 bis 0,9% Wasser und kann über ein Ventil 13 und eine Leitung 14 weggeführt werden.

Ein Teil des Kondensats kann über eine Leitung 12 an den Kopf des Rektifikationsaufsatzes 8 zurückgegeben werden.

Die spezifisch leichtere Oberphase des Absetzbehälters 11 enthält im wesentlichen das Reaktionswasser und ist mit Monochloressigsäureester gesättigt (2% im Falle des Ethylesters).

Nach Phasentrennung wird die wäßrige Phase einer Wasserdampfdestillation unterworfen und auf Monochloressigsäureester soweit abgereichert, daß sie problemlos verworfen werden kann.

Die erhaltenen Wasserdampfdestillate werden gesammelt und anschließend auf Monochloressigsäureester verarbeitet.

Als Veresterungskatalysatoren eignen sich die dem Fachmann geläufigen, z.B. aromatische Sulfonsäuren. Nach beendeter Veresterung wird nach Abkühlen des Reaktionsgemisches auf Umgebungstemperatur der Katalysator durch Waschen mit wäßriger Bicarbonatlösung und Wasser entfernt; der erhaltene Monochloressigsäureester wird über ein Ventil 15 und eine Leitung 16 abgezogen.

Zur kontinuierlichen Durchführung des Verfahrens der Erfindung nach dem Fließschema gemäß Fig. 2 verwendet man eine Kolonne mit einem Reaktionsteil 20, der mit Glockenböden ausgestattet ist, und aufgesetztem Kolonnenkopf 21. Die mit dem Katalysator versehene Monochloressigsäure-Schmelze wird über eine Leitung 22 einem Wärmeaustauscher 23 zugeführt und dort auf 135 bis 160 °C erwärmt und in die Kolonne eingegeben. Stromabwärts zum Aufgabeort der Monochloressigsäure wird über eine Leitung 24 in einem Wärmeaustauscher 25 überhitzter Alkanoldampf (135 bis 160 °C) eingeblasen, so das das Alkanol im Gegenstromprinzip mit der Monochloressigsäure reagiert. Das gebildete, daß Reaktionswasser enthaltende Azeotrop wird über den Kolonnenkopf 21 und eine Leitung 26 mit dazwischengeschaltetem Wärmeaustauscher 27 abgenommen und einem Absetzbehälter 28 zugeführt. Ein Teil der Unterphase in dem Absetzbehälter wird über eine Leitung 29 an den Kolonnenkopf als Rücklauf zurückgegeben; der Hauptteil der Unterphase wird über eine Leitung 30 weggeführt. Die obere Phase wird, wie oben erläutert, durch Wasserdampfdestillation aufgearbeitet. In dem Reaktionsteil 20 der Kolonne erfolgt unterhalb der Eintrittsstelle für den Alkanoldampf der Restumsatz und die Abreicherung an niedrigsiedenden Bestandteilen.

Die Prozeßwärme wird durch indirekte Wärmezufuhr in der Kolonne und/oder durch extern angeordnete, nicht gezeigte Wärmeaustauscher, die im Umlauf betrieben werden, zugeführt.

Die Abnahme des Monochloressigsäureesters erfolgt aus dem Kolonnensumpf über eine Leitung 32 und ein dazwischengeschaltetes Ventil 33; ein Teil des Produktes wird über eine Leitung 34 und einen Wärmeaustauscher 35 an den Kolonnensumpf zurückgegeben. Der Hauptteil des Produktes wird über eine Leitung 36 weggeführt und mit der Unterphase des Absetzbehälters 28 vereinigt.

Das Verfahren der Erfindung wird weiterhin durch das folgende Ausführungsbeispiel näher erläutert.

Beispiel.

In einem 1,6m³-Reaktor wurden 945 kg (10,0 kMol) Monochloressigsäure und 18,0 kg (0,1 kMol) p-Toluolsulfonsäure aufgeschmolzen. Die Schmelze wurde durch indirekte Beheizung bei Normaldruck auf 140°C gebracht. Bei dieser Temperatur wurden über eine Lanze in Bodennähe des Reaktors insgesamt 465 kg (10,09 kMol) Ethanol so eindosiert, daß nach Einsetzen der Destillation das Destillat (Ober- und Unterphase) weitgehend ethanolfrei sind. Die Zugabedauer betrug ca. 1 Stunde.

Das Kondensat trennte spontan in zwei Phasen auf. Während nach Trennung der Phasen die wäßrige Oberphase (182 kg) mittels Wasserdampfdestillation für die Entsorgung von Monochloressigsäureester wurde, wurde die untere Phase als Wertprodukt entnommen (213,2 kg). Parallel dazu wurde der Reaktorinhalt nach Abkühlung auf 25°C einmal mit 80 kg 10 %-iger wäßriger Kaliumcarbonatlösung und danach mit 80 kg Wasser säurefreigewaschen. Es wurden 996,4 kg Monochloressigsäureethylester (99,9 %d.Th.) erhalten. Das Produkt wies eine gaschromatografische Reinheit von 99.1 % auf.

## Ansprüche

1. Verfahren zur Herstellung von Estern der Monochloressigsäure mit $C_{1-4}$-Alkanolen durch Veresterung der freien Säure mit den Alkoholen in Gegenwart von Veresterungskatalysatoren, dadurch gekennzeichnet, daß man den Alkohol der katalysatorhaltigen Schmelze der Monochloressigsäure unter Entfernung des Reaktionswassers mit solcher Geschwindigkeit zugibt, daß das sich bildende Reaktionswasser zusammen mit Anteilen des enstandenen Chloressigsäureesters als praktisch alkoholfreies binäres Azeotrop abdestilliert wird.

2. Verfahren nach Anspruch 1, dadurch gekennzeichnet, daß man die Veresterung unter gleichzeitiger Abtrennung der alkoholfreien dampfförmigen wasserhaltigen Phase bei Temperaturen oberhalb 100°C, vorzugsweise im Bereich von etwa 120 - 170°C und insbesondere im Bereich von etwa 135 - 160°C durchführt.

3. Verfahren nach Ansprüchen 1 und 2, dadurch gekennzeichnet, daß man im Druckbereich von etwa 0,3 - 5 bar, insbesondere im Bereich des Normaldrucks arbeitet.

4. Verfahren nach Ansprüchen 1 bis 3, dadurch gekennzeichnet, daß man - bezogen auf die Gesamtumsetzung - mit etwa äquimolaren Mengen an Monochloressigsäure zu $C_{1-4}$-Alkohol arbeitet, die bevorzugt im Bereich von 1 : 1-1,05 liegen.

5. Verfahren nach Ansprüchen 1 bis 4, dadurch gekennzeichnet, daß man den Gehalt des Reaktionsgemisches an freiem Alkohol während der Umsetzung auf Werte von maximal etwa 1 Gew.-% begrenzt.

6. Verfahren nach Ansprüchen 1 bis 5, dadurch gekennzeichnet, daß man das destillativ abgetrennte binäre Azeotrop unter Phasentrennung kondensiert, die untere Phase als Wertprodukt abtrennt und aus der oberen wäßrigen Phase bevorzugt durch erneute partielle Destillation wenigstens anteilsweise die dort enthaltenen Restmengen an Chloressigsäureester gewinnt.

7. Verfahren nach Ansprüchen 1 bis 6, dadurch gekennzeichnet, daß man absatzweise, bevorzugt aber kontinuierlich wie folgt arbeitet: Gegenstromführung der katalysatorhaltigen Schmelze der Monochloressigsäure und des Alkohols in einer Reaktionskolonne bei Einspeisung der Schmelze im Kopfbereich, Abziehen des wasserhaltigen binären Azeotrops am Kolonnenkopf, bevorzugt unter Rückführung eines Anteils des nach Kondensation aus dem Azeotrop gewonnenen Chloressigsäureesters auf den Kolonnenkopf und Gewinnung der Hauptmenge des Chloressigsäureesters am anderen Ende der Reaktionskolonne.

8. Verfahren nach Ansprüchen 1 bis 7, dadurch gekennzeichnet, daß man den Alkohol etwa auf Reaktionstemperatur vorerhitzt in die Schmelzphase einführt.

9. Verfahren nach Ansprüchen 1 bis 8, dadurch gekennzeichnet, daß man als Alkohol Methanol, oder Ethanol, vorzugsweise Ethanol, einsetzt.

Fig. 1

D 7771 EP

Fig. 2

D 7771 *EP*